(19) Europäisches Patentamt
European Patent Office
Office européen des brevets

(11) **EP 1 840 213 A1**

(12) **EUROPEAN PATENT APPLICATION**
published in accordance with Art. 158(3) EPC

(43) Date of publication:
**03.10.2007 Bulletin 2007/40**

(21) Application number: **05820352.2**

(22) Date of filing: **21.12.2005**

(51) Int Cl.:
*C12N 15/09* (2006.01)   *A61K 41/00* (2006.01)
*A61P 43/00* (2006.01)   *C07K 14/435* (2006.01)
*C07K 19/00* (2006.01)   *C07D 405/10* (2006.01)

(86) International application number:
**PCT/JP2005/023508**

(87) International publication number:
**WO 2006/068187 (29.06.2006 Gazette 2006/26)**

(84) Designated Contracting States:
**CH DE FR GB LI**

(30) Priority: **21.12.2004   JP 2004368946**
**21.12.2004   JP 2004368947**

(71) Applicants:
• **Riken**
**Wako-shi, Saitama 351-0198 (JP)**
• **Japan Science and Technology Agency**
**Kawaguchi-shi,**
**Saitama 332-0012 (JP)**

(72) Inventors:
• **NAGAI, Takeharu,**
**4-502, Chuo 2 Syukusya**
**Sapporo-shi, Hokkaido 0600806 (JP)**
• **MIYAWAKI, Atsushi**
**Wako-shi, Saitama 3510112 (JP)**

(74) Representative: **polypatent**
**An den Gärten 7**
**51491 Overath (DE)**

(54) **TARGET PHYSIOLOGICAL FUNCTION INACTIVATOR USING PHOTOSENSITIZER-LABELED FLUORESCENT PROTEIN**

(57)    An object of the present invention is to provide a method of generating reactive oxygen species in a light irradiation-dependent manner, so as to inactivate any target physiological function. The present invention provides a target physiological function inactivator which consists of a photosensitizer-labeled fluorescent protein, wherein fluorescence resonance energy transfer (FRET) from the fluorescent protein to the photosensitizer occurs as a result of light irradiation, so that the photosensitizer can be excited to generate reactive oxygen species.

**EP 1 840 213 A1**

**Description**

Technical Field

The present invention relates to a target physiological function inactivator using generation of reactive oxygen species via fluorescence resonance energy transfer from a fluorescent protein to a photosensitizer. Moreover, the present invention relates to a split fluorescent protein that is labeled with a photosensitizer, and a method of inactivating a target physiological function using the structure-function complementarity of the above protein.

Background Art

Green fluorescent protein (GFP) derived from *Aequorea victoria,* a jellyfish, has many purposes in biological systems. Recently, various GFP mutants have been produced based on the random mutagenesis and semi-rational mutagenesis, wherein a color is changed, a folding property is improved, luminance is enhanced, or pH sensitivity is modified. Fluorescent proteins such as GFP are fused with other proteins by gene recombinant technique, and monitoring of the expression and transportation of the fusion proteins is carried out.

One of the most commonly used types of GFP mutant is Yellow fluorescent protein (YFP). Among Aequorea-derived GFP mutants, YFP exhibits the fluorescence with the longest wavelength. The values $\varepsilon$ and $\Phi$ of the majority of YEPs are 60,000 to 100,000 $M^{-1}cm^{-1}$ and 0.6 to 0.8, respectively (Tsien, R. Y. (1998). Ann. Rev. Biochem. 67, 509-544). These values are comparable to those of the general fluorescent group (fluorescein, rhodamine, etc.). Moreover, Cyan fluorescent protein (CFP) is another example of GFP mutants. Among such Cyan fluorescent proteins, ECFP (enhanced cyan fluorescent protein) has been known. Furthermore, Red fluorescent protein (RFP) has been isolated from sea anemone *(Discoma sp.),* and among such red fluorescent proteins, DasRed has been known. Thus, 4 types of fluorescent proteins including green, yellow, cyan and red fluorescent proteins, have been developed one after another, and their spectrum range has been significantly extended.

In order to analyze the function of a biomolecule, a method of biochemically inactivating the molecular function is effective. That is to say, the function of a target molecule is inhibited, and the thus inhibited target molecule is compared with a case where the above molecule normally functions, so as to analyze in detail the function of the target molecule. However, it has been known that the functions of biomolecules are not always uniform in a cell, but that a specific biomolecule efficiently exhibits its function at a specific local site in the cell. Accordingly, when a target molecule functions under a specific communication, by inactivating such a function of the target molecule at the site where it functions, the function of the target molecule in a living cell can be clarified. As a method of inactivating a target molecule in a temporally and spatially controlled manner, a method of laser inactivation of molecules (chromopphore-assisted laser inactivation; CALI) is described in Daniel G. Jay, Proc. Natl. Acad. Sci. USA, Vol. 85, pp. 5454-5458, 1988, for example. However, it has also been difficult for this method to efficiently inactivate a target molecule in a living cell in a temporally and spatially controlled manner.

Disclosure of Invention

Object to be Solved by the Invention

It is an object of the present invention to provide a method of generating reactive oxygen species in a light irradiation-dependent manner, so as to inactivate any given target substance (target physiological function).

Means for Solving the Object

As a result of intensive studies directed towards achieving the aforementioned object, the present inventors have found that excitation light for fluorescent protein is applied to a photosensitizer-labeled fluorescent protein, and fluorescence resonance energy transfer from the fluorescent protein to the photosensitizer is thereby allowed to occur, so that the photosensitizer can be excited to generate reactive oxygen species. Moreover, the present inventors have also found that the C-terminal fragment of the photosensitizer-labeled fluorescent protein is introduced into a cell that expresses any given target protein genetically ligated to the N-terminal fragment of the fluorescent protein, so that the structure of the fluorescent protein is reconstituted in the cell, and thereafter, fluorescence resonance energy transfer from the reconstituted fluorescent protein to the photosensitizer is utilized to generate reactive oxygen species from the photosensitizer, thereby inactivating any given target protein. The present invention has been completed based on such findings.

That is to say, the present invention provides a target physiological function inactivator which consists of a photosensitizer-labeled fluorescent protein, wherein fluorescence resonance energy transfer (FRET) from the fluorescent protein to the photosensitizer occurs as a result of light irradiation, so that the photosensitizer can be excited to generate reactive oxygen species.

Preferably, at least a portion of the fluorescence spectrum of the fluorescent protein is overlapped with a portion of the absorption spectrum of the photosensitizer.

Preferably, the fluorescent protein is a GFP mutant.

Preferably, the fluorescent protein is a CFP mutant or an EGFP mutant.

Preferably, the fluorescent protein is: a fluorescent protein produced by substituting serine at position 72 with alanine, serine at position 175 with glycine, and alanine at position 206 with lysine, of ECFP; or a fluorescent protein produced by substituting threonine at position 203 with isoleucine of EGFP.

Fluorescence resonance energy transfer (FRET) from the fluorescent protein to the photosensitizer occurs at an efficiency of preferably 80% or more, and more preferably 90% or more.

Preferably, the photosensitizers bind to amino acid residues corresponding to the amino acid residue at position 6 and/or the amino acid residue at position 229 of CFP.

Preferably, the photosensitizer is eosin.

In another aspect, the present invention provides a method of generating reactive oxygen species in a light irradiation-dependent manner, using the aforementioned target physiological function inactivator of the present invention, so as to inactivate a target physiological function.

Preferably, inactivation of the target physiological function is inactivation of a protein.

In a further aspect, the present invention provides a method of inactivating a target physiological function, which comprises: a step of introducing into a cell that expresses a fused protein consisting of either the N-terminal fragment or the C-terminal fragment of a fluorescent protein and any given protein, a labeled protein produced by labeling the other fragment of the fluorescent protein with a photosensitizer, so as to reconstitute a fluorescent protein in the cell; and a step of applying light to said reconstituted fluorescent protein, so as to cause fluorescence resonance energy transfer (FRET) from the fluorescent protein to the photosensitizer, thereby exciting the photosensitizer to generate reactive oxygen species.

Preferably, at least a portion of the fluorescence spectrum of the fluorescent protein is overlapped with a portion of the absorption spectrum of the photosensitizer.

Preferably, the fluorescent protein is CFP or a mutant thereof.

Preferably, the photosensitizer is eosin.

Preferably, either one amino acid sequence of two types of amino acid sequences that interact with each other is further fused with the fused protein consisting of either the N-terminal fragment or the C-terminal fragment of the fluorescent protein and any given protein, and the other amino acid sequence of the above two types of amino acid sequences that interact with each other is further fused with the labeled protein produced by labeling the other fragment of the fluorescent protein with the photosensitizer.

In a further aspect, the present invention provides a kit for carrying out the aforementioned method of inactivating a target physiological function of the present invention, which comprises either the N-terminal fragment or the C-terminal fragment of a fluorescent protein or a gene encoding thereof, and a labeled protein produced by labeling the other fragment of the fluorescent protein with a photosensitizer.

In a further aspect, the present invention provides a kit for carrying out the aforementioned method of inactivating a target physiological function of the present invention, which comprises a cell that expresses a fused protein consisting of either the N-terminal fragment or the C-terminal fragment of a fluorescent protein and any given protein, and a labeled protein produced by labeling the other fragment of the fluorescent protein with a photosensitizer.

Best Mode for Carrying out the Invention

The embodiments of the present invention will be described in detail below.

(1) Target physiological function inactivator using generation of reactive oxygen species via fluorescence resonance energy transfer from fluorescent protein to photosensitizer

In a first embodiment, the present invention relates to a target physiological function inactivator which consists of a photosensitizer-labeled fluorescent protein, wherein fluorescence resonance energy transfer (FRET) from the fluorescent protein to the photosensitizer occurs as a result of light irradiation, so that the photosensitizer can be excited to generate reactive oxygen species.

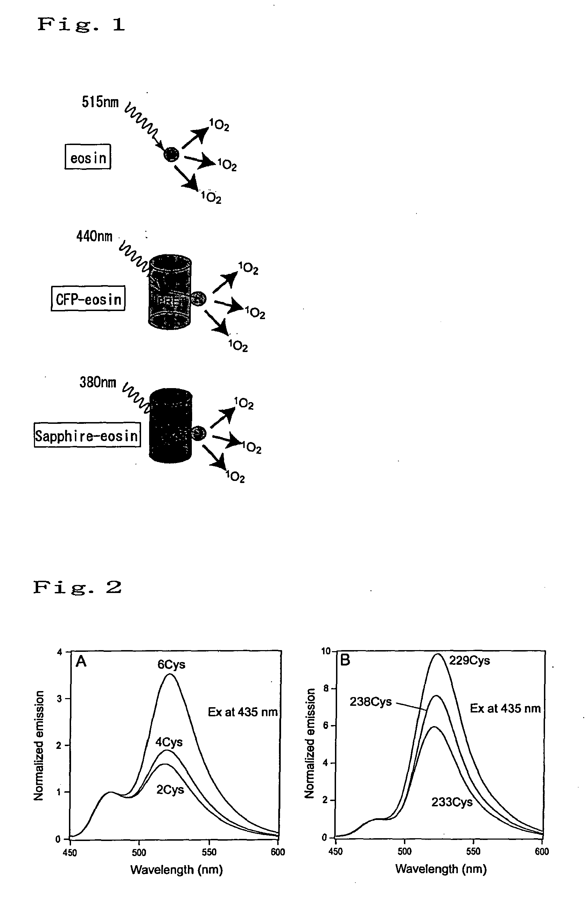

The outline of the method of the present invention is shown in Figure 1. As shown in Figure 1, in the present invention, fluorescence resonance energy transfer (FRET) from a fluorescent protein (a GFP mutant, etc.) to a photosensitizer is used to generate reactive oxygen species. In the example as shown in Figure 1, eosin is used as a photosensitizer, and CFP or Sapphire which is a GFP mutant is used as a fluorescent protein. Any type of combination of a fluorescent protein (a GFP mutant, etc.) and a photosensitizer can be used, as long as the fluorescence spectrum of the fluorescent protein is overlapped with the absorption spectrum of the photosensitizer to a moderate degree. In the present invention, the FRET efficiency from the fluorescent protein to the photosensitizer is preferably 80% or more. When such FRET efficiency is less than 80%, photobleaching of the fluorescent protein occurs due to irradiation with strong light, and further, reactive oxygen species is not generated. Thus, it is not favorable.

As a fluorescent protein used in the present invention, any type of protein can be used, as long as it is able to emit fluorescence as a result of irradiation with excitation light, and it allows a photosensitizer as described later to cause fluorescence resonance energy transfer. The fluorescent protein used in the present invention acts as a donor fluorescent protein in the aforementioned fluorescence resonance energy transfer.

Examples of a fluorescent protein that can be used in the present invention include a cyan fluorescent protein (CFP), a yellow fluorescent protein (YFP), a green fluorescent protein (GFP), a red fluorescent protein (RFP), a blue fluorescent protein (BFP), and a mutant thereof.

The expression "a cyan fluorescent protein, a yellow fluorescent protein, a green fluorescent protein, a red fluorescent protein, a blue fluorescent protein, or a mutant thereof" is used in the present specification not only to mean known fluorescent proteins, but also to include all the mutants thereof (e.g. ECFP, EYFP, EGFP, ERFP, EBFP, etc. obtained by enhancing the fluorescence intensity of each of the aforementioned fluorescent proteins). For example, the gene of such a green fluorescent protein has been isolated and sequenced (Prasher, D. C. et al. (1992), "Primary structure of the Aequorea victoria green fluorescent protein," Gene 111 : 229-233). The amino acid sequences of a large number of other fluorescent proteins or mutants thereof have also been reported. Such amino acid sequences are described in Roger Y. Tsien, Annu. Rev. Biochem. 1998. 67: 509-44, and the cited documents thereof, for example. As such a green fluorescent protein (GFP), a yellow fluorescent protein (YFP), or a mutant thereof, those derived from *Aequorea coerulescens* (e.g. *Aequorea victoria)* can be used, for example.

The nucleotide sequences of genes encoding the fluorescent proteins used in the present invention have been known. As such genes encoding the above fluorescent proteins, commercially available products can also be used. For example, the EGFP vector, EYFP vector, ECFP vector, and EBFP vector, which are commercially available from Clontech, can be used as such gene products.

Moreover, fluorescent proteins obtained by introducing a novel mutation into amino acids of various types of known fluorescent proteins as described above can also be used. A method of introducing a desired mutation into any given nucleic acid sequence has been known to persons skilled in the art. For example, DNA comprising a mutation can be constructed using, as appropriate, known techniques such as sited-directed mutagenesis, PCR using degenerate oligonucleotides, or a technique of exposing cells containing nucleic acids to a mutation-inducing agent or radioactive ray. Such known techniques are described, for example, in Molecular Cloning: A laboratory Manual, 2nd Ed., Cold Spring Harbor Laboratory, Cold Spring Harbor, N. Y., 1989; and Current Protocols in Molecular Biology, Supplement 1 to 38, John Wiley & Sons (1987-1997).

Any type of photosensitizer can be used in the present invention, as long as it is able to generate reactive oxygen species (singlet oxygen) when it is excited by fluorescence resonance energy transfer from the aforementioned fluorescent protein, and as long as the fluorescence spectrum of the fluorescent protein is moderately overlapped with the absorption spectrum of the photosensitizer so that FRET occurs between the photosensitizer and the aforementioned fluorescent protein.

Specific examples of such a photosensitizer include eosin, fluorescein, methylene blue, rose bengal, acid red, protoporphyrin, and hematoporphyrin.

Labeling of a fluorescent protein with a photosensitizer can be carried out by any given method. For example, one or several amino acid residues in the amino acid sequence of a fluorescent protein have previously been substituted with cysteine. Thereafter, such a fluorescent protein having a cysteine residue(s) is allowed to react with a maleimidized photosensitizer such as eosin maleimide, so as to produce a photosensitizer-labeled fluorescent protein.

When a fluorescent protein is labeled with a photosensitizer according to the aforementioned method, a position to be labeled can be arbitrarily selected by selecting a position into which a cysteine residue is to be introduced. In the present invention, it is preferable that the aforementioned position to be labeled be selected such that a high fluorescence resonance energy transfer (FRET) efficiency can be achieved. A fluorescence resonance energy transfer (FRET) efficiency from a fluorescent protein to a photosensitizer is preferably 80% or more, more preferably 90% or more, and further more preferably 93% or more.

In the case of using eosin as a photosensitizer for example, a high FRET efficiency can be achieved when photosensitizers bind to amino acid residues that correspond to the amino acid residue at position 6 and/or the amino acid residue at position 229 of CFP. Accordingly, in a preferred embodiment of the present invention, amino acids that correspond to the amino acid at position 6 and/or the amino acid at position 229 of CFP are substituted with cysteine, and photosensitizers can be then allowed to bind to such cysteine residues.

In the present invention, excitation light for fluorescent proteins is applied to a photosensitizer-labeled fluorescent protein, so that fluorescence resonance energy transfer from the fluorescent protein to the photosensitizer is allowed to occur. The wavelength of the excitation light used herein can be selected, as appropriate, depending on the type of the fluorescent protein used. The irradiation time of such excitation light is not particularly limited. Such excitation light can be applied for approximately several milliseconds to 10 minutes, for example.

The photosensitizer-labeled fluorescent protein used in the present invention causes fluorescence resonance energy transfer (FRET) from the fluorescent protein to the photosensitizer as a result of light irradiation. The photosensitizer is thereby excited to generate reactive oxygen species. Thus, the above photosensitizer-labeled fluorescent protein can be used as a target physiological function inactivator. That is to say, reactive oxygen species can be generated by introducing the photosensitizer-labeled fluorescent protein into a cell by methods such as microinjection, or by directly injecting the above fluorescent protein into living tissues, followed by irradiation with excitation light. As a result of such generation of reactive oxygen species, a target substance (a protein, etc.) existing around the reactive oxygen species becomes inactivated. As a result, a target physiological function becomes inactivated.

Moreover, another protein may be further fused with the photosensitizer-labeled fluorescent protein used in the present invention. The type of another protein to be fused is not particularly limited. Examples of such a protein to be fused include a protein localized in a cell, a protein specific for a cell organella, and a targeting signal (e.g. a nuclear localization signal, a mitochondrial presequence). Otherwise, it is also possible to fuse a protein for inactivating functions or a protein interacting with such a protein for inactivating functions, with the photosensitizer-labeled fluorescent protein.

(2) Method of inactivating target physiological function using structure-function complementarity of split fluorescent protein labeled with photosensitizer

In a second embodiment, the present invention relates to a method of inactivating a target physiological function, which comprises: a step of introducing into a cell that expresses a fused protein consisting of either the N-terminal fragment or the C-terminal fragment of a fluorescent protein and any given protein, a labeled protein produced by labeling the other fragment of the fluorescent protein with a photosensitizer, so as to reconstitute a fluorescent protein in the cell; and a step of applying light to the above reconstituted fluorescent protein, so as to cause fluorescence resonance energy transfer (FRET) from the fluorescent protein to the photosensitizer, thereby exciting the photosensitizer to generate reactive oxygen species.

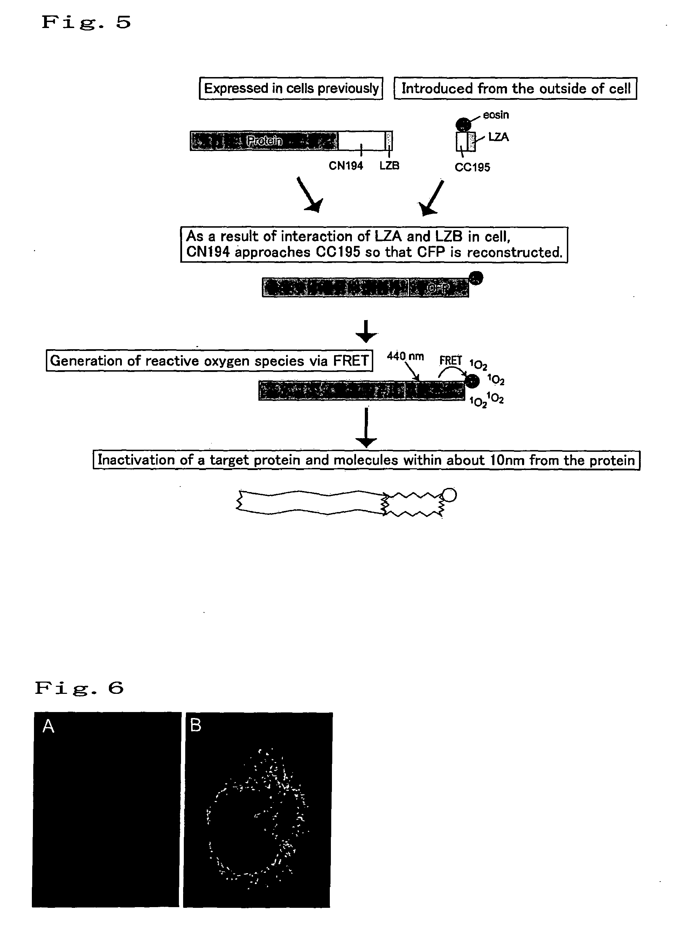

The outline of the method of the present invention is shown in Figure 5. Figure 5 shows a general outline of a method of inactivating a physiological function utilizing reconstitution of split CFP and fluorescence resonance energy transfer from the protein to a photosensitizing dye. The term "Protein" is used to mean a protein to be inactivated, or a sequence to be localized in a cell organella. CC195-LZA, which has been labeled with a dye (photosensitizer), may be introduced into a cell according to microinjection or a beads load method. Otherwise, a protein transduction domain peptide such as TAT or 9R may be ligated to the above protein, and the thus ligated protein may be then added to a medium or flowing blood, so as to introduce it into the cell.

As a fluorescent protein used in the present invention, any type of protein can be used, as long as it is able to emit fluorescence as a result of irradiation with excitation light, and as long as it allows a photosensitizer as described later to cause fluorescence resonance energy transfer. The fluorescent protein used in the present invention acts as a donor fluorescent protein in the aforementioned fluorescence resonance energy transfer.

Examples of a fluorescent protein that can be used in the present invention include a cyan fluorescent protein (CFP), a yellow fluorescent protein (YFP), a green fluorescent protein (GFP), a red fluorescent protein (RFP), a blue fluorescent protein (BFP), and a mutant thereof.

The expression "a cyan fluorescent protein, a yellow fluorescent protein, a green fluorescent protein, a red fluorescent protein, a blue fluorescent protein, or a mutant thereof" is used in the present specification not only to mean known fluorescent proteins, but also to include all the mutants thereof (e.g. ECFP, EYFP, EGFP, ERFP, EBFP, etc. obtained by enhancing the fluorescence intensity of each of the aforementioned fluorescent proteins). For example, the gene of such a green fluorescent protein has been isolated and sequenced (Prasher, D. C. et al. (1992), "Primary structure of the Aequorea victoria green fluorescent protein," Gene 111 : 229-233). The amino acid sequences of a large number of other fluorescent proteins or mutants thereof have also been reported. Such amino acid sequences are described in Roger Y. Tsien, Annu. Rev. Biochem. 1998. 67: 509-44, and the cited documents thereof, for example. As such a green fluorescent protein (GFP), a yellow fluorescent protein (YFP), or a mutant thereof, those derived from *Aequorea coerulescens* (e.g. *Aequorea victoria)* can be used, for example.

The nucleotide sequences of genes encoding the fluorescent proteins used in the present invention have been known. As such genes encoding the above fluorescent proteins, commercially available products can also be used. For example, the EGFP vector, EYFP vector, ECFP vector, and EBFP vector, which are commercially available from Clontech, can be used as such gene products.

Moreover, fluorescent proteins obtained by introducing a novel mutation into amino acids of various types of known fluorescent proteins as described above can also be used. A method of introducing a desired mutation into any given nucleic acid sequence has been known to persons skilled in the art. For example, DNA comprising a mutation can be constructed using, as appropriate, known techniques such as sited-directed mutagenesis, PCR using degenerate oligonucleotides, or a technique of exposing cells containing nucleic acids to a mutation-inducing agent or radioactive ray. Such known techniques are described, for example, in Molecular Cloning: A laboratory Manual, 2nd Ed., Cold Spring Harbor Laboratory, Cold Spring Harbor, N. Y., 1989; and Current Protocols in Molecular Biology, Supplement 1 to 38, John Wiley & Sons (1987-1997).

In the present invention, the fluorescent protein is divided into an N-terminal side and a C-terminal side before use. That is, either the N-terminal fragment or the C-terminal fragment of the fluorescent protein is fused with another protein (e.g. a target protein to be inactivated, a sequence to be localized in a cell organella, etc.), and the thus fused protein is further fused with an amino acid sequence that interacts with another amino acid sequence used to reconstitute the fluorescent protein in a cell. Thereafter, the thus fused protein is allowed to express in a cell in the aforementioned form in advance.

As an example, in Figure 5, the N-terminal fragment of a fluorescent protein (indicated as "CN194" in Figure 5) has previously been allowed to express in a cell in the form of a protein that has been fused with another protein (indicated as "Protein" in Figure 5) and an amino acid sequence (LZB) for reconstituting the fluorescent protein in the cell.

The type of "another protein," which is fused with either the N-terminal fragment or the C-terminal fragment of the fluorescent protein, is not particularly limited. Examples of such "another protein" include a protein localized in a cell, a protein specific for a cell organella, and a targeting signal (e.g. a nuclear localization signal, a mitochondrial presequence, etc.). Otherwise, it is also possible that a protein for inactivating functions or a protein interacting with such a protein for inactivating functions be fused with such "another protein."

The aforementioned fused protein is allowed to express in a cell according to a common method. That is to say, DNA encoding the fused protein is prepared, and the DNA is then incorporated into a suitable expression vector. Thereafter, the obtained recombinant expression vector is introduced into cells so as to carry out genetic transformation. A suitable expression vector may be selected, as appropriate, depending on the type of a cell used as a host.

The type of a cell used as a host is not particularly limited. Bacterial cells, mammalian cells, yeast cells, or other types of cells can be used. Examples of bacterial cells include: Gram-positive bacteria such as Bacillus or Streptomyces; and Gram-negative bacteria such as *Escherichia coli.* Such bacterial cells may be transformed by the protoplast method or known methods using competent cells. Examples of mammalian cells include HEK293 cells, HeLa cells, COS cells, BHK cells, CHL cells, and CHO cells. Such mammalian cells may be transformed by electroporation, the calcium phosphate method, lipofection, or the like, for example. Examples of yeast cells include cells belonging to genus Saccharomyces or genus Schizosaccharomyces. Specific examples include *Saccharomyces cerevislae* and *Saccharomyces kluyveri.* Examples of a method of introducing a recombinant vector into a yeast host include electroporation, the spheroplast method, and the lithium acetate method.

On the other hand, the other fragment (the N-terminal fragment or the C-terminal fragment) of the fluorescent protein is labeled with a photosensitizer. Thereafter, it is fused with an amino acid sequence that interacts with the amino acid sequence fused with either the N-terminal fragment or the C-terminal fragment of the fluorescent protein, which is useful for reconstitution of the fluorescent protein in a cell. As an example, in Figure 5, in the case of the C-terminal fragment of the fluorescent protein (indicated as CC195 in Figure 5), a protein which was fused with eosin acting as a photosensitizer and an amino acid sequence (LZA) used for reconstitution of the fluorescent protein in a cell are introduced into a cell from the outside. The above protein may be introduced into the cell from the outside by microinjection or a beads load method. Otherwise, a protein transduction domain peptide such as TAT or 9R may be ligated to the above protein, and the thus ligated protein may be then added to a medium or flowing blood, so as to introduce it into the cell.

As stated above, either the N-terminal fragment or the C-terminal fragment of the fluorescent protein has previously been allowed to express in a cell, and thereafter, the other fragment of the fluorescent protein is introduced into the cell from the outside. Thus, amino acid sequences, which have been fused with the two above fragments, interact with each other in the cell. As a result, the N-terminal fragment of the fluorescent protein and the C-terminal fragment thereof get closer to each other, so that the fluorescent protein can be reconstituted in the cell.

Any type of photosensitizer can be used in the present invention, as long as it is able to generate reactive oxygen species (singlet oxygen) when it is excited by fluorescence resonance energy transfer from the aforementioned fluorescent protein, and as long as the fluorescence spectrum of the fluorescent protein is moderately overlapped with the absorption spectrum of the photosensitizer so that FRET occurs between the photosensitizer and the aforementioned fluorescent protein.

Specific examples of such a photosensitizer include eosin, fluorescein, methylene blue, rose bengal, acid red, protoporphyrin, and hematoporphyrin.

Labeling of the N-terminal fragment or the C-terminal fragment of a fluorescent protein with a photosensitizer can be carried out by any given method. For example, one or several amino acid residues in the amino acid sequence of the N-terminal fragment or C-terminal fragment of a fluorescent protein have previously been substituted with cysteine. Thereafter, such a fluorescent protein having a cysteine residue(s) is allowed to react with a maleimidized photosensitizer such as eosin maleimide, so as to produce a photosensitizer-labeled fluorescent protein.

In the present invention, excitation light for fluorescent proteins is applied to a fluorescent protein that has been reconstituted in a cell (wherein this fluorescent protein has been labeled with a photosensitizer), so as to cause fluorescence resonance energy transfer from the fluorescent protein to the photosensitizer. The wavelength of the excitation light used herein can be selected, as appropriate, depending on the type of the fluorescent protein used. The irradiation time of such excitation light is not particularly limited. Such excitation light can be applied for approximately several milliseconds to 10 minutes, for example.

In the present invention, reactive oxygen species can be generated by introducing the other fragment of a fluorescent protein, which has been labeled with a photosensitizer, into a cell by methods such as microinjection, or by directly injecting the other fragment into living tissues, followed by irradiation with excitation light. Thus, as a result of such generation of reactive oxygen species, a target substance (a protein, etc.) existing around the reactive oxygen species becomes inactivated. As a result, the target physiological function of the cells existing in a region wherein reactive oxygen species is generated becomes inactivated.

The present invention will be specifically described in the following examples. However, these examples are not intended to limit the scope of the present invention.

EXAMPLES

Example A-1: Construction of CFP and Sapphire mutants

First, in order to improve the maturation efficiency of the ECFP protein and to prevent multimer formation, there was constructed a gene encoding mSECFP-72A, wherein serine at position 72 was substituted for alanine, serine at position 175 was substituted for glycine, and alanine at position 206 was substituted for lysine. Using ECFP/pRSETB as a template, and also using the following three primers, mutagenesis was carried out according to the method described in a publication (Sawano and Miyawaki, Nucleic Acids Res. 28: E78, 2000):

5'-CAGTGCTTCGCCCGCTACCCC-3' (SEQ ID NO: 1);
5'-GAGGACGGCGGCGTGCAGCTC-3' (SEQ ID NO: 2); and
5'-TACCAGTCCAAGCTGAGCAAA-3' (SEQ ID NO: 3).

Sapphire was constructed by substituting threonine at position 203 of EGFP with isoleucine. For substitution of the amino acid, the same above method was applied using the following primer:

5'-TACCTGAGCATCCAGTCCGCC-3' (SEQ ID NO: 4).

Subsequently, in order to substitute the amino acids at positions 2, 4, 6, 229, 233, and 238 of both mSECFP-72A and Sapphire with cysteine, PCR was carried out using mSECFP-72A/pRSETB or Sapphire/pRSETB as a template, and also using the following primer sets.

Primer set used in amplification of mSECFP-2C/72A and Sapphire-2C:

5'-ATTGGATCCCGCCTGCAAGGGCGAGGAGCTGTTC-3' (SEQ ID NO: 5); and
5'-ATTGAATTCTTACTTGTACAGCTCGTCCATG-3' (Primer A) (SEQ ID NO: 6)

Primer set used in amplification of mSECFP-4C/72A and Sapphire-4C:

5'-ATTGGATCCCGGCTGCGAGGAGCTGTTCACCGGG-3' (SEQ ID NO: 7); and Primer A.

Primer set used in amplification of mSECFP-6C/72A and Sapphire-6C:

5'-ATTGGATCCCGGCTGCCTGTTCACCGGGGTGGTG-3' (SEQ ID NO: 8); and Primer A.

Primer set used in amplification of mSECFP-72A/229C and Sapphire-229C:

5'-CGGGGTACCATGGTGAGCAAGGGCGAG-3' (Primer B) (SEQ ID NO: 9); and
5'-GCAGAATTCTTAGCAGTACAGCTCGTCCTAGCC-3' (SEQ ID NO: 10).

Primer set used in amplification of mSECFP-72A/229C and Sapphire-233C:

Primer B; and

5'-GCAGAATTCTTAGCAGCCGAGAGTGATCCCGGC-3' (SEQ ID NO: 11).

Primer set used in amplification of mSECFP-72A/229C, Sapphire-238C:

Primer B; and

5'-GCAGAATTCTTAGCACCCGGCGGCGGTCACGAAC-3' (SEQ ID NO: 12).

Each PCR product was cleaved with the restriction enzymes BamHI and EcoRI, and the cleaved portion was then inserted into the BamHI-EcoRI of pRSETB, so as to construct mSECFP-2C/72A-pRSETB, mSECFP-4C/72A-pRSETB, mSECFP-6C/72A-pRSETB, mSECFP-72A/229C-pRSETB, mSECFP-72A/233C-pRSETB, mSECFP-72A/238C-pRSETB, Sapphire-2C-pRSETB, Sapphire-4C-pRSETB, Sapphire-6C-pRSETB, Sapphire-229C-pRSETB, Sapphire-233C-pRSETB, and Sapphire-238C-pRSETB.

Example A-2: Preparation of CFP mutant proteins

In order to generate proteins such as mSECFP-2C/72A, mSECFP-4C/72A, mSECFP-6C/72A, mSECFP-72A/229C, mSECFP-72A/233C, mSECFP-72A/238C, Sapphire-2C, Sapphire-4C, Sapphire-6C, Sapphire-229C, Sapphire-233C, and Sapphire-238C in *Escherichia coli*, *Escherichia coli* (JM109 DE3) was transformed with 10 ng each of mSECFP-2C/72A-pRSETB, mSECFP-4C/72A-pRSETB, mSECFP-6C/72A-pRSETB, mSECFP-72A/229C-pRSETB, mSECFP-72A/233C-pRSETB, mSECFP- 72A/ 238C- pRSETB, Sapphire-2C- pRSETB, Sapphire- 4C- pRSETB, Sapphire- 6C-pRSETB, Sapphire- 229C- pRSETB, Sapphire- 233C-pRSETB, and Sapphire- 238C- pRSETB. Each of the obtained transformants was cultured for 1 day in an LB plate that contained 100 mg/ml ampicillin. Thereafter, a single *Escherichia coli* colony was picked up, and it was then inoculated into 200 ml of LB medium that contained 100 mg/ml ampicillin, followed by shaking culture at 20°C for 4 days. Thereafter, a cell mass was recovered by centrifugation, and it was then suspended in 10 ml of PBS (-). Thereafter, the cell mass was disintegrated using a French press. 2 ml of Ni- NTA agarose was added to a supernatant, from which the residue had been removed by centrifugation, and the mixture was shaken for 1 hour. A protein adsorbed on the Ni- NTA agarose was filled into a column, and it was then washed with 5 ml of PBS (-), followed by elution with 1 ml of 100 mM imidazole/PBS (-). Thereafter, imidazole was removed from the resultant by the gel filtration method, so as to obtain a purified protein solution.

Example A-3: Labeling of CFP mutant protein with dye

100 $\mu$l of the purified protein solution was dissolved in 500 $\mu$l of PBS(-) that contained 1 mM TCEP, and the obtained solution was then incubated at room temperature for 30 minutes. Thereafter, eosin maleimide or fluorescein maleimide (both of which were available from Molecular Probe) was added to the resultant to a final concentration of 0.3 mM, and the obtained mixture was then reacted in a dark place at room temperature for 2 hours. Thereafter, unreacted dye was removed by the gel filtration method, so as to obtain a dye-labeled protein solution. The concentration of the protein was determined by the Bradford method.

Example A-4: Measurement of spectrum of dye-labeled CFP mutant protein

Eosin has weak fluorescence. Thus, using a fluorescein-labeled protein, a site having a high FRET efficiency was first examined. 5 mg of a fluorescein-labeled CFP mutant protein was dissolved in 1 ml of PBS(-). Thereafter, the fluorescence spectrum obtained with excitation light at 435 nm was measured using a fluorospectrophotometer (HITACHI F-2500). As a result, it was revealed that a high FRET efficiency can be obtained when the amino acid at position 6 on the N-terminal side and the amino acid at position 229 on the C-terminal side are labeled with dye (Figure 2). In Figure 2, A represents a fluorescence spectrum obtained when the amino acids at positions 2, 4, and 6 on the N-terminal side were substituted for cysteine and the protein was then labeled with fluorescein maleimide. An excitation wavelength of 435 nm was used. In Figure 2, B represents a fluorescence spectrum obtained when the amino acids at positions 229, 233, and 238 on the C-terminal side were substituted for cysteine and the protein was then labeled with fluorescein maleimide. An excitation wavelength of 435 nm was used. Even in the case of labeling the above protein with eosin, the same tendency was observed.

In order to further improve such FRET efficiency, amino acids, at which the maximum FRET efficiency had been obtained on the N- and C-terminal sides, were labeled with eosin, and the spectrum was measured before and after labeling. In addition, the FRET efficiency was calculated based on the intensity ratio of the fluorescence peaks (480 nm) of the CFP mutant, Sapphire in the presence or absence of labeling. The following formula was used for calculation:

$$E_T = 1 - (F_{DA}/F_D)$$

$E_T$ represents FRET efficiency, $F_{DA}$ represents the fluorescence intensity at 480 nm of the CFP mutant protein that has been labeled with the dye, and $F_D$ represents the fluorescence intensity at 480 nm of the CFP mutant protein that has not been labeled with the dye.

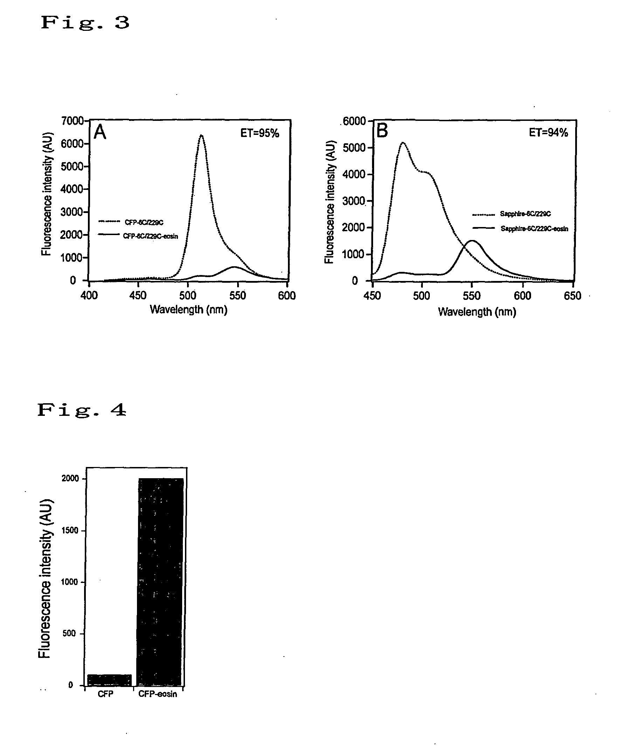

Figure 3 shows a change in the spectra obtained before and after labeling and the FRET efficiency. In Figure 3, A represents Sapphire, and B represents CFP. In both cases, a broken line indicates the spectrum of an unlabeled fluorescent protein, and a solid line indicates the spectrum of a labeled fluorescent protein.

Example A-5: Measurement of amount of reactive oxygen species generated

A possibility that eosin-labeled CFP generates singlet oxygen in a light irradiation-dependent manner was examined. For the measurement of the amount of singlet oxygen, the Singlet Oxygen Sensor Green Reagent (SOSGR, Molecular Probe) was used as a probe. SOSGR is a nonfluorescent reagent. However, when it specifically reacts with singlet oxygen, it emits fluorescence of 525 nm. 1 $\mu$g of eosin-unlabeled or eosin-labeled mSECFP-6C/229C (which were CFP and CFP-eosin, respectively) was dissolved in 15 $\mu$l of PBS, and SOSGR was then added to the obtained solution to a final concentration of 66.7 $\mu$M. 5 $\mu$l of the obtained mixture was transferred into 2 wells of a Terasaki plate. Thereafter, 8 mW of 430-nm laser was applied to one of the two wells for 1 minute. Thereafter, the total amount of solution was diluted with 300 $\mu$l of PBS(-), and the fluorescence intensity at 525 nm obtained by excitation at 505 nm was then measured using a fluorospectrophotometer (HITACHI F-2500). The value of the sample that had not been irradiated with the laser was subtracted from the value of the sample that had been irradiated with the laser, and the obtained value was defined as a relative amount of reactive oxygen species generated. From Figure 4, it is found that eosin-labeled mSECFP-6C/229C generated a considerable amount of singlet oxygen as a result of irradiation of light at 430 nm. Eosin does not have absorption at 430 nm, and thus it is understand that the above result was obtained as a result that energy had been efficiently transferred from CFP excited with the light at 430 nm to eosin via FRET.

Example B-1: Construction of CN194/pcDNA3 and CC195/pcDNA3

Using mSECFP-72A/229C-pRSETB as a template, PCR was carried out with the following primer sets. Primer set used in amplification of CN194:

    5'-CCCAAGCTTCCACCATGGTGAGCAAGGGCGAGGAG-3' (SEQ ID NO: 13); and
    5'-ATTGGATCCCAGCACGGGGCCGTCGCC-3' (SEQ ID NO: 14).

Primer set used in amplification of CC 195:

    5'-CCCAAGCTTCCACCATGCTGCCCGACAACCACTACCTG-3' (SEQ ID NO: 15); and
    5'-ATTGGATCCCTTGTACAGCTCGTCCATGCC-3' (SEQ ID NO: 16).

The PCR product was cleaved with the restriction enzymes HindIII and BamHI, and the cleaved portion was then inserted into the HindIII-BamHI of pcDNA3 (Invitrogen), so as to construct CN194/pcDNA3 and CC195/pcDNA3.

Example B-2: Construction of CN194-LZB/pcDNA3 and CC195-LZA/pcDNA3

ACID-p1 (LZA) and BASE-p1 (LZB) that form a heterodimeric coiled coil structure (Erin K et al., Current Biology 3, 658-667, 1993) were amplified by PCR using the following synthetic oligonucleotides and primer sets.

LZA synthetic oligonucleotide:

5'-GGA GGC GCC CAG CTA GAA AAG GAG CTG CAA GCC CTG GAG AAG GAG AAC GCC CAG CTC GAA TGG GAG CTC CAG GCC CTG GAG AAG GAG CTG GCC CAG AAG TAA-3' (SEQ ID NO: 17).

Primer set used in amplification of LZA:

    5'-ATT GGA TCC GGA GGC GCC CAG CTA GAAAAG-3' (SEQ ID NO: 18); and

5'- ATT GAA TTC TTA CTT CTG GGC CAG CTC CTT C-3' (SEQ ID NO: 19).

LZB synthetic oligonucleotide:

    5'-GGA GGC GCC CAG CTC AAG AAG AAG CTG CAA GCC CTG AAG AAG AAG AAC GCC CAG CTC AAG TGG AAG CTC CAG GCC CTG AAG AAG AAG CTG GCC CAG AAG TAA-3' (SEQ ID NO: 20).

Primer set used in amplification of LZB:

    5'-ATT GGA TCC GGA GGC GCC CAG CTC AAG AAG-3' (SEQ ID NO: 21); and
    5'- ATT GAA TTC TTA CTT CTG GGC CAG CTT CTT C-3' (SEQ ID NO: 22).

The PCR products of LZA and LZB were cleaved with the restriction enzymes BamHI and EcoRI, and the cleaved portions were then inserted into the BamHI-EcoRI site of CC195/pcDNA3 and that of CN194/pcDNA3, respectively, so as to construct CC195-LZA/pcDNA3 and CN194-LZB/pcDNA3.

Example B-3: Construction of hOC/pcDNA3

The mitochondrial localization sequence of human ornithine carbamylase (hOC) was amplified by the overlap PCR method using the following synthetic oligonucleotides and primer set.
hOC-N synthetic oligonucleotide:

    5'-ACC ATG CTG TTC AAC CTG AGG ATC CTG CTG AAC AAC GCC GCC TTC AGG AAC GGC CAC AAC TTC ATG GTG AGG-3' (SEQ ID NO: 23).

hOC-C synthetic oligonucleotide:

    5'-CCC CTG CAC CTT GTT CTG CAG GGG CTG GCC GCA CCT GAA GTT CCT CAC CAT GAA GTT GTG GCC GTT-3' (SEQ ID NO: 24).

Primer set used in amplification of hOC-N:

    5'-CCCAAGCTTCCACCATGCTGTTCAACCTGAGGATC-3' (SEQ ID NO: 25); and
    5'-CGCAAGCTTTCGGCCGCCCTGCACCTTGTTCTGCAGGGGCTG-3' (SEQ ID NO: 26).

The PCR product of hOC was cleaved with the restriction enzymes HindIII and NotI, and the cleaved portion was then inserted into the HindIII-NotI site of pcDNA3, so as to construct hOC/pcDNA3.

Example B-4: Construction of hOC-CN194-LZB/pcDNA3 and hOC-CC195-LZA/pcDNA3

Using CC195-LZA/pcDNA3 and CN194-LZB/pcDNA3 as templates, CN194-LZB and CC195-LZA were amplified by PCR with the following primer sets.

Primer set used in amplification of CN194-LZB:

5'-CCCAAGCTTGCGGCCGCATGGTGAGCAAGGGCGAGGAG-3' (SEQ ID NO: 27); and

5'-CCGCTCGAGTTACTTCTGGGCCAGCTTCTTC-3' (SEQ ID NO: 28).

Primer set used in amplification of CC195-LZA:

5'-CCCAAGCTTGCGGCCGCATGCTGCCCGACAACCACTAC-3' (SEQ ID NO: 29); and

5'-CCGCTCGAGTTACTTCTGGGCCAGCTCCTTC-3' (SEQ ID NO: 30).

The PCR products of CN194-LZB and CC195-LZA were cleaved with the restriction enzymes NotI and XhoI, and each of the cleaved portions was inserted into the NotI-XhoI site of hOC/pcDNA3, so as to construct hOC-CN194-LZB/pcDNA3 and hOC-CC195-LZA/pcDNA3, respectively.

Example B-5: Construction of hOC-CC195-LZA/pET23a

Using hOC-CC195-LZA/pcDNA3 as a template, hOC-CC195-LZA was amplified by PCR with the following primer set.

Primer set used in amplification of hOC-CC195-LZA:

5'-GGAATTCCATATGCTGTTCAACCTGAGGATC-3' (SEQ ID NO: 31); and
5'-CCGCTCGAGCTTCTGGGCCAGCTCCTTC-3' (SEQ ID NO: 32).

The PCR product was cleaved with the restriction enzymes NdeI and XhoI, and the cleaved portion was then inserted into the NdeI-XhoI site of pET23a (Novagen), so as to construct hOC-CC195-LZA/pET23a.

Example B-6: Preparation of dye-labeled hOC-CC195-LZA protein

*Escherichia coli* (JM109 DE3) was transformed with 10 ng of hOC-CC195-LZA/pET23a. The obtained transformant was cultured for 1 day in an LB plate that contained 100 μg/ml ampicillin. Thereafter, a single *Escherichia coli* colony was picked up, and it was then inoculated into 200 ml of LB medium that contained 100 μg/ml ampicillin, followed by shaking culture at 20°C for 4 days. Thereafter, a cell mass was recovered by centrifugation, and it was then suspended in 10 ml of PBS (-). Thereafter, the cell mass was disintegrated using a French press. The residue was removed by centrifugation, so as to produce a roughly purified hOC-CC195-LZA protein solution. Subsequently, TCEP was added to 2.5 ml of the roughly purified hOC-CC195-LZA protein solution to a final concentration of 1 mM. The obtained mixture was incubated at room temperature for 30 minutes. Thereafter, eosin maleimide or fluorescein maleimide (both of which were available from Molecular Probe) was added to the resultant to a final concentration of 0.3 mM, and the obtained mixture was then reacted in a dark place at room temperature for 2 hours. After completion of the reaction, 2 ml of Ni-NTA agarose was added to the reaction product, and the obtained mixture was then shaken for 1 hour. A protein adsorbed on the Ni-NTA agarose was filled into a column, and it was then washed with 5 ml of PBS(-), followed by elution with 1 ml of 100 mM imidazole/PBS(-). Thereafter, imidazole was removed from the resultant by the gel filtration method, so as to obtain a purified protein solution.

Example B-7: Reconstitution of fluorescent protein in mitochondria of HeLa cells and disruption of mitochondria by light irradiation

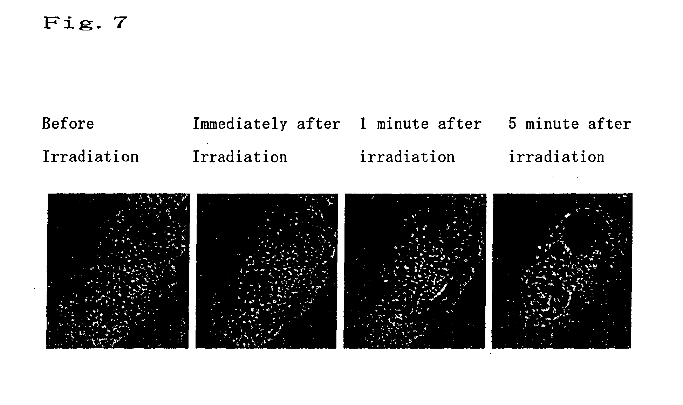

1 x 10^5 HeLa S3 cells cultured on a 35-mm glass bottom plate were transfected with 1 μg of hOC-CN194-LZB/pcDNA3 by lipofection. 24 hours later, unlabeled hOC-CC195-LZA was introduced into the HeLa cells by a beads load method. 4 hours later, the presence or absence of fluorescence was observed. FV1000 confocal laser microscope manufactured by Olympus Corp. was used as a microscope, and PLANApo x60 NA1.2 Water was used as objective lens. The protein was excited with laser light (laser power: 15%) of 458-nm line (maximum output: 3 mW) of a multi-argon laser, so as to obtain fluorescence at 470 to 560 nm.
No fluorescence was observed in the HeLa cells that expressed only hOC-CN194-LZB, into which unlabeled hOC-CC195-LZA had not been introduced. In contrast, in the case of the HeLa cells into which unlabeled hOC-CC195-LZA had been introduced, emission of fluorescence from the mitochondria was observed (Figure 6). In Figure 6, A represents the fluorescent image of the HeLa cells that expressed only hOC-CN194-LZB, and B represents the fluorescent image obtained 4 hours after introduction of hOC-CC195-LZA into the HeLa cells that expressed only hOC-CN194-LZB. The results show that CN194-LZB and CC195-LZA, which had been transferred to the matrices of mitochondria by hOC, interacted with each other via LZB and LZA, so that the structure of CFP could be recovered. Subsequently, eosin-labeled hOC-CC195-LZA was introduced into the cells, and 4 hours later, the cells were observed in the same above manner. As a result, emission of fluorescence from mitochondria was observed. A change in cells having mitochondria with fluorescent property occurring after irradiation of the cells with laser of 405 nm (output: 10 mW) was observed by lapse of time (Figure 7). As a result, it was found that the cells that had been irradiated with light of 405 nm died due to necrosis several minutes after the light irradiation. On the other hand, the cells, into which unlabeled hOC-CC195-LZA had been introduced, did not die even after the same light irradiation. These results suggested that energy transferred from CFP excited with laser of 405 nm to eosin, so as to generate singlet oxygen, resulting in inactivation of several functions of mitochondria.

Industrial Applicability

The present invention made it possible to generate reactive oxygen species in a light irradiation-dependent manner, thereby inactivating any given target substance (target physiological function). In the present invention, when light is applied, a target substance can be inactivated by reactive oxygen species only in a place to which the light is applied. There are cases where the function of a biomolecule is generally allowed to express at a specific site wherein the physiological function is exhibited, or at a specific time in a stage of generation of individuals. Thus, the method of the present invention capable of time- and space-specifically inactivating a target substance is useful for the analysis of the function of such a biomolecule.

Brief Description of the Drawings

Figure 1 shows the outline of the method of the present invention.
Figure 2 shows fluorescence spectra obtained when fluorescein has been introduced into different sites of CFP.
Figure 3 shows fluorescence spectra and FRET efficiency that are obtained when positions 6 and 229 of each of Sapphire and CFP were substituted for cysteine, and the protein was labeled with eosin maleimide.
Figure 4 shows generation of reactive oxygen species (singlet oxygen) due to excitation of a photosensitizer via FRET.
Figure 5 shows the outline of the method of the present invention.
Figure 6 shows reconstitution of split CFP in mitochondria.
Figure 7 shows the results regarding induction of necrosis (cellular necrosis) due to disruption of mitochondria.

**Claims**

1. A target physiological function inactivator which consists of a photosensitizer-labeled fluorescent protein, wherein fluorescence resonance energy transfer (FRET) from the fluorescent protein to the photosensitizer occurs as a result of light irradiation, so that the photosensitizer can be excited to generate reactive oxygen species.

2. The target physiological function inactivator of claim 1 wherein at least a portion of the fluorescence spectrum of the fluorescent protein is overlapped with a portion of the absorption spectrum of the photosensitizer.

3. The target physiological function inactivator of claim 1 or 2 wherein the fluorescent protein is a GFP mutant.

4. The target physiological function inactivator of any of claims 1 to 3 wherein the fluorescent protein is a CFP mutant or an EGFP mutant.

5. The target physiological function inactivator of any of claims 1 to 4 wherein the fluorescent protein is: a fluorescent protein produced by substituting serine at position 72 with alanine, serine at position 175 with glycine, and alanine at position 206 with lysine, of ECFP; or a fluorescent protein produced by substituting threonine at position 203 with isoleucine of EGFP.

6. The target physiological function inactivator of any of claims 1 to 5 wherein fluorescence resonance energy transfer (FRET) from the fluorescent protein to the photosensitizer occurs at an efficiency of 80% or more.

7. The target physiological function inactivator of any of claims 1 to 5 wherein fluorescence resonance energy transfer (FRET) from the fluorescent protein to the photosensitizer occurs at an efficiency of 90% or more.

8. The target physiological function inactivator of any of claims 1 to 7 wherein the photosensitizers bind to amino acid residues corresponding to the amino acid residue at position 6 and/or the amino acid residue at position 229 of CFP.

**9.** The target physiological function inactivator of any of claims 1 to 8 wherein the photosensitizer is eosin.

**10.** A method of generating reactive oxygen species in a light irradiation-dependent manner, using the target physiological function inactivator of any of claims 1 to 9, so as to inactivate a target physiological function.

**11.** The method of claim 10 wherein inactivation of the target physiological function is inactivation of a protein.

**12.** A method of inactivating a target physiological function, which comprises: a step of introducing into a cell that expresses a fused protein consisting of either the N-terminal fragment or the C-terminal fragment of a fluorescent protein and any given protein, a labeled protein produced by labeling the other fragment of the fluorescent protein with a photosensitizer, so as to reconstitute a fluorescent protein in the cell; and a step of applying light to said reconstituted fluorescent protein, so as to cause fluorescence resonance energy transfer (FRET) from the fluorescent protein to the photosensitizer, thereby exciting the photosensitizer to generate reactive oxygen species.

**13.** The method of claim 12 wherein at least a portion of the fluorescence spectrum of the fluorescent protein is overlapped with a portion of the absorption spectrum of the photosensitizer.

**14.** The method of claim 12 or 13 wherein the fluorescent protein is CFP or a mutant thereof.

**15.** The method of any of claims 12 to 14 wherein the photosensitizer is eosin.

**16.** The method of any of claims 12 to 15 wherein either one amino acid sequence of two types of amino acid sequences that interact with each other is further fused with the fused protein consisting of either the N-terminal fragment or the C-terminal fragment of the fluorescent protein and any given protein, and the other amino acid sequence of the above two types of amino acid sequences that interact with each other is further fused with the labeled protein produced by labeling the other fragment of the fluorescent protein with the photosensitizer.

**17.** A kit for carrying out the method of any of claims 12 to 16, which comprises either the N-terminal fragment or the C-terminal fragment of a fluorescent protein or a gene encoding thereof, and a labeled protein produced by labeling the other fragment of the fluorescent protein with a photosensitizer.

**18.** A kit for carrying out the method of any of claims 12 to 16, which comprises a cell that expresses a fused protein consisting of either the N-terminal fragment or the C-terminal fragment of a fluorescent protein and any given protein, and a labeled protein produced by labeling the other fragment of the fluorescent protein with a photosensitizer.

## Fig. 1

## Fig. 2

Fig. 3

Fig. 4

Fig. 5

| Expressed in cells previously | Introduced from the outside of cell |

eosin

Protein
CN194    LZB

LZA
CC195

As a result of interaction of LZA and LZB in cell,
CN194 approaches CC195 so that CFP is reconstructed.

CFP

Generation of reactive oxygen species via FRET    440 nm    FRET $^1O_2$
$^1O_2$
$^1O_2$ $^1O_2$

Inactivation of a target protein and molecules within about 10nm from the protein

Fig. 6

A    B

Fig. 7

Before            Immediately after   1 minute after    5 minute after
Irradiation       Irradiation         irradiation       irradiation

## INTERNATIONAL SEARCH REPORT

| | International application No. |
|---|---|
| | PCT/JP2005/023508 |

A.  CLASSIFICATION OF SUBJECT MATTER
*C12N15/09*(2006.01), *A61K41/00*(2006.01), *A61P43/00*(2006.01), *C07K14/435*
(2006.01), *C07K19/00*(2006.01), *C07D405/10*(2006.01)

According to International Patent Classification (IPC) or to both national classification and IPC

B.  FIELDS SEARCHED

Minimum documentation searched (classification system followed by classification symbols)
A61K41/00, A61P43/00, C07D405/10, C07K14/435, C07K19/00, C12N15/09

Documentation searched other than minimum documentation to the extent that such documents are included in the fields searched
Jitsuyo Shinan Koho            1922-1996   Jitsuyo Shinan Toroku Koho   1996-2006
Kokai Jitsuyo Shinan Koho     1971-2006   Toroku Jitsuyo Shinan Koho   1994-2006

Electronic data base consulted during the international search (name of data base and, where practicable, search terms used)
CA/BIOSIS/MEDLINE/WPIDS(STN)

C.  DOCUMENTS CONSIDERED TO BE RELEVANT

| Category* | Citation of document, with indication, where appropriate, of the relevant passages | Relevant to claim No. |
|---|---|---|
| Y<br>A | SUZKI Miho et al., Protease-sensitive signalling by chemically engineered intramolecular fluorescent resonance energy transfer mutants of green fluorescent protein Biochimica et Biophysica Acta (2004), 1679, 222-229 | 1-11<br>12-18 |
| Y<br>A | GANTCHEV Tsvetan G. et al., Catalase inactivation following photosensitization with tetrasulfonated metallophthalocyanines Photochemistry and Photobiology (1995), 62(1), 123-134 | 1-11<br>12-18 |

☒  Further documents are listed in the continuation of Box C.       ☐  See patent family annex.

| | | |
|---|---|---|
| * | Special categories of cited documents: | "T"  later document published after the international filing date or priority date and not in conflict with the application but cited to understand the principle or theory underlying the invention |
| "A" | document defining the general state of the art which is not considered  to be of particular relevance | |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| 27 March, 2006 (27.03.06) | 04 April, 2006 (04.04.06) |

| Name and mailing address of the ISA/<br>     Japanese Patent Office | Authorized officer |
|---|---|
| Facsimile No. | Telephone No. |

Form PCT/ISA/210 (second sheet) (April 2005)

**INTERNATIONAL SEARCH REPORT**

| International application No. |
| --- |
| PCT/JP2005/023508 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y<br>A | SCHNEIDER J. Edward Jr et al., Methylene blue and rose Bengal photoinactivation of RNA bacteriophage:comparative studies of 8-oxoguanine formation in isolated RNA Archives of Biochemistry and Biophysics (1993), 301(1), 91-97 | 1-11<br>12-18 |
| Y | SAWANO Asako et al., Directed evolution of green fluorescent protein by a new versatile PCR strategy for site-directed and semi-random mutagenesis Nucleic Acids Research(2000), 28(16), e78 | 5 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TSIEN, R. Y.** *Ann. Rev. Biochem.,* 1998, vol. 67, 509-544
- **DANIEL G. JAY.** *Proc. Natl. Acad. Sci. USA,* 1988, vol. 85, 5454-5458
- **PRASHER, D. C. et al.** Primary structure of the Aequorea victoria green fluorescent protein. *Gene,* 1992, vol. 111, 229-233
- **ROGER Y. TSIEN.** *Annu. Rev. Biochem.,* 1998, vol. 67, 509-44
- Molecular Cloning: A laboratory Manual. Cold Spring Harbor Laboratory, 1989
- Current Protocols in Molecular Biology. John Wiley & Sons, 1987, 1-38
- **ERIN K et al.** *Current Biology,* 1993, vol. 3, 658-667